# EUROPEAN PATENT SPECIFICATION

(11) **EP 2 521 562 B1**
(45) Date of publication and mention of the grant of the patent: **18.09.2019**
(21) Application number: 11700973.8
(22) Date of filing: 10.01.2011
(51) Int. Cl.: A61K 38/16, A61P 31/16

(54) **EV576 FOR USE IN THE TREATMENT OF VIRAL INFECTIONS OF THE RESPIRATORY TRACT**
EV576 ZUR BEHANDLUNG VON VIRUSINFEKTIONEN DER ATEMWEGE
EV576 POUR UTILISATION DANS TRAITEMENT D'INFECTIONS VIRALES DES VOIES RESPIRATOIRES

(30) Priority: 25.03.2010 GB 201005071; 08.01.2010 GB 201000318
(43) Date of publication of application: 14.11.2012
(73) Proprietor: Volution Immuno Pharmaceuticals SA, 3461-1211 Geneve 3 (CH)
(72) Inventor: WESTON-DAVIES, Wynne, London W1T 4EU (GB)
(74) Representative: Goodfellow, Hugh Robin
(86) International application number: PCT/GB2011/000022
(87) International publication number: WO 2011/083317

(56) References cited:
- WO-A1-2007/117241
- WO-A2-2008/029169
- WO-A2-2009/098454

### SEQUENCE LISTING

<110> VARLEIGH IMMUNOPHARMACEUTICALS LIMITED
<120> METHOD OF TREATMENT
<130> P054003WO
<150> GB1000318.4
   <151> 2010-01-08
<150> GB1005071.4
   <151> 2010-03-25
<160> 2
<170> SeqWin99, version 1.02
<210> 1
   <211> 507
   <212> DNA
   <213> Ornithodoros moubata
<400> 1
   atgctggttt tggtgaccct gattttctcc ttttctgcga acatcgcata tgctgacagc   60
   gaaagcgact gcactggaag cgaacctgtt gacgccttcc aagctttcag tgagggcaaa  120
   gaggcatatg tcctggtgag gtccacggat cccaaagcga gggactgctt gaaaggagaa  180
   ccagccggag aaaagcagga caacacgttg ccggtgatga tgacgtttaa gaatggcaca  240
   gactgggctt caaccgattg gacgtttact ttggacggcg caaaggtaac ggcaaccctt  300
   ggtaacctaa cccaaaatag ggaagtggtc tacgactcgc aaagtcatca ctgccacgtt  360
   gacaaggtcg agaaggaagt tccagattat gagatgtgga tgctcgatgc gggagggctt  420
   gaagtggaag tcgagtgctg ccgtcaaaag cttgaagagt tggcgtctgg caggaaccaa  480
   atgtatcccc atctcaagga ctgctag                                      507
<210> 2
   <211> 168
   <212> PRT
   <213> Ornithodoros moubata
<400> 2
   Met Leu Val Leu Val Thr Leu Ile Phe Ser Phe Ser Ala Asn Ile Ala
   1               5                   10                  15
   Tyr Ala Asp Ser Glu Ser Asp Cys Thr Gly Ser Glu Pro Val Asp Ala
               20                  25                  30
   Phe Gln Ala Phe Ser Glu Gly Lys Glu Ala Tyr Val Leu Val Arg Ser
           35                  40                  45
   Thr Asp Pro Lys Ala Arg Asp Cys Leu Lys Gly Glu Pro Ala Gly Glu
       50                  55                  60
   Lys Gln Asp Asn Thr Leu Pro Val Met Met Thr Phe Lys Asn Gly Thr
   65                  70                  75                  80
   Asp Trp Ala Ser Thr Asp Trp Thr Phe Thr Leu Asp Gly Ala Lys Val
                   85                  90                  95
   Thr Ala Thr Leu Gly Asn Leu Thr Gln Asn Arg Glu Val Val Tyr Asp
               100                 105                 110
   Ser Gln Ser His His Cys His Val Asp Lys Val Glu Lys Glu Val Pro
           115                 120                 125
   Asp Tyr Glu Met Trp Met Leu Asp Ala Gly Gly Leu Glu Val Glu Val
       130                 135                 140
   Glu Cys Cys Arg Gln Lys Leu Glu Glu Leu Ala Ser Gly Arg Asn Gln
   145                 150                 155                 160
   Met Tyr Pro His Leu Lys Asp Cys
                   165

## Description

### Field of the Invention

The present invention relates to treating and preventing the inflammatory effects of viral infection of the upper and lower respiratory tracts, including infection by SARS coronovirus (SARS), pandemic Influenza A H5N1 (avian influenza) and influenza A H1N1 (swine 'flu).

### Background to the Invention

The mortality associated with SARS and pandemic influenza is linked to rapidly progressive respiratory failure causing acute lung injury (ALI) or acute respiratory distress syndrome (ARDS). In some cases, multi-organ failure is also a feature. In the case of pandemic H5N1 influenza, the mortality due to respiratory and multi-organ failure is around 60%. The primary lung pathology of fatal H1N1 influenza has recently been described and is characterised by necrotising alveolitis and dense neutrophil infiltration [1].

Originally, it was assumed that respiratory failure associated with SARS and pandemic influenza was due to rapid viral replication leading to cytolytic destruction of target cells of the respiratory tract, such as alveolar epithelial cells, or to escape of the virus to tissues and organs remote from the respiratory system, such as the central nervous system. Recent evidence has shown, however, that the development of respiratory failure is not, in fact, associated with high viral titres. Investigators have instead found that respiratory failure is associated with significant elevation of pro-inflammatory cytokines such as TFNα and IFNβ. This has led experts to propose that the pathogenesis of these complications is inappropriate stimulation of the innate immune system triggering a so-called 'cytokine storm'[2,3].

Current treatments for respiratory failure involve increasing the patient's oxygen levels using an oxygen mask, mechanical oxygenation using a ventilator or, in the most severe case, extracorporeal membrane oxygenation (ECMO) which involves circulating the patient's blood outside the body and adding oxygen to it artificially.

There is a great need for agents that improve upon the currently available treatments for the respiratory failure caused by the inflammatory effects of viral infection of the respiratory tract.

### Summary of the Invention

The invention provides a therapeutically or prophylactically effective amount of an agent that inhibits the classical complement pathway, the alternative complement pathway and the lectin complement pathway for use in treating or preventing the inflammatory effects of viral infection of the respiratory tract, wherein the agent is (i) a protein comprising or consisting of amino acids 19 to 168 or amino acids 1 to 168 of the amino acid sequence SEQ ID NO:2; (ii) a homologue of a protein as defined in (i) comprising or consisting of a sequence with at least 90% identity to the sequence of amino acids 19 to 168 or amino acids 1 to 168 of the amino acid sequence SEQ ID NO:2; or (iii) a fragment of a protein as defined in (i) above or of a homologue as defined in (ii) above wherein the fragment binds complement C5 and/or LTB4.

The complement system is an essential part of the body's natural defence mechanism against foreign invasion and is also involved in the inflammatory process. More than 30 proteins in serum and at the cell surface are involved in complement system function and regulation. Recently it has become apparent that, as well as the ∼35 known components of the complement system which may be associated with both beneficial and pathological processes, the complement system itself interacts with at least 85 biological pathways with functions as diverse as angiogenesis, platelet activation, glucose metabolism and spermatogenesis

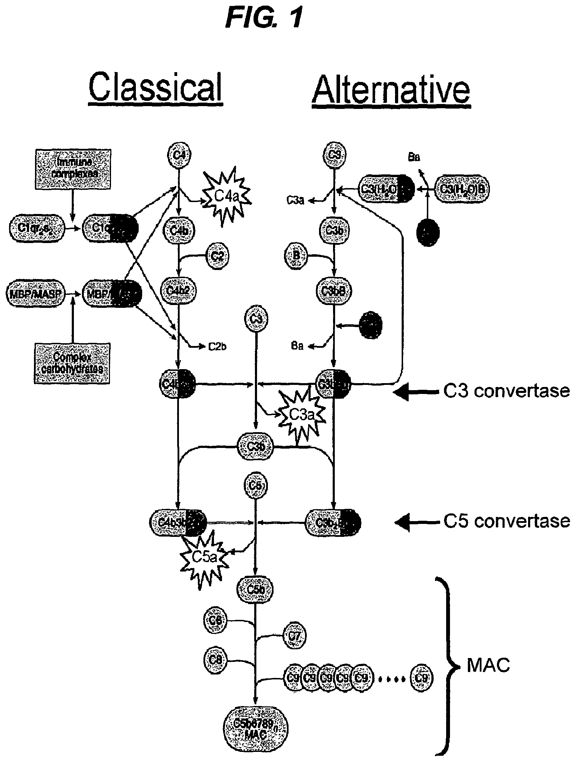

The complement system is activated by the presence of foreign antigens. Three activation pathways exist: (1) the classical pathway which is activated by IgM and IgG complexes or by recognition of carbohydrates; (2) the alternative pathway which is activated by non-self surfaces (lacking specific regulatory molecules) and by bacterial endotoxins; and (3) the lectin pathway which is activated by binding of manna-binding lectin (MBL) to mannose residues on the surface of a pathogen. The three pathways comprise parallel cascades of events that result in the production of complement activation through the formation of similar C3 and C5 convertases on cell surfaces resulting in the release of acute mediators of inflammation (C3a and C5a) and formation of the membrane attack complex (MAC). The parallel cascades involved in the classical and alternative pathways are shown in Figure 1.

The complement system is recognised as being an early activator of innate immune responses initiating many inflammatory cascades. However it has not previously been implicated as being a cause of the respiratory complications of viral infection of the respiratory system. Surprisingly, the data presented in the current application show for the first time that an agent that inhibits the alternative, classical and lectin complement pathways reduces the inflammatory effects of viral infection of the respiratory tract.

Reduction of the inflammatory effects of viral infection of the respiratory tract may be assessed by reduction in inflammatory cytokines and/or neutrophils in a subject suffering from such a viral infection. In one aspect of the invention, administration of the agent that inhibits the alternative, classical and lectin complement pathways may thus reduce levels of inflammatory cytokines, such as CXCL2, IL-1β, and/or IL-6, in a subject suffering from viral infection of the respiratory tract compared to an untreated subject. Administration of the agent that inhibits the alternative, classical and lectin complement pathways to a subject suffering from a viral infection of the respiratory tract may also reduce levels of neutrophils compared to an untreated subject. Cytokine levels and neutrophil levels may, for example, be assessed in bronchoalveolar lavage (BAL) fluid from the subject.

In one aspect of the invention, the agent, as defined in the claims, may bind complement C5. The agent may act to prevent the cleavage of complement C5 by C5 convertase into complement C5a and complement C5b-9. The agent may act to reduce C5a levels in a subject suffering from a viral infection of the respiratory tract, for example in the BAL fluid from such a subject, compared to an untreated subject. Surprisingly, the data presented in the current application show for the first time that there is a significant increase in C5a in BAL fluid following viral infection of the respiratory tract.

The complement C5 protein, also referred to herein as C5, is cleaved by the C5 convertase enzyme, itself formed from C3a, an earlier product of the alternative pathway (Figure 1). The products of this cleavage include an anaphylatoxin C5a and a lytic complex C5b - 9 also known as membrane attack complex (MAC). C5a is a highly reactive peptide implicated in many pathological inflammatory processes including neutrophil and eosinophil chemotaxis, neutrophil activation, increased capillary permeability and inhibition of neutrophil apoptosis [4].

MAC is associated with other important pathological processes including rheumatoid arthritis [5;6], proliferative glomerulonephritis [7], idiopathic membranous nephropathy [8], proteinurea [9], demyelination after acute axonal injury [10] and is also responsible for acute graft rejection following xenotransplantation [11].

C5a has become a target of particular interest in the field of complement-associated disorders [12]. Although C5a has many well-recognised pathological associations, the effects of its depletion in humans appear to be limited. Monoclonal antibodies and small molecules that bind and inhibit C5a or C5a receptors have been developed to treat various autoimmune diseases. These molecules do not, however, prevent the release of MAC.

In contrast, the agent used in the current invention inhibits both the formation of C5a peptide and the MAC. Since C5 is a late product of the classical and alternative complement pathways, inhibition of C5 is less likely to be associated with risks of concomitant infection that exist when targeting earlier products in the cascade [13].

The ability of an agent to bind C5 may be determined by standard *in vitro* assays known in the art, for example by western blotting following incubation of the protein on the gel with labelled C5. Preferably, the agent according to the invention binds C5 with an IC₅₀ of less than 0.2 mg/ml, preferably less than 0.1 mg/ml, preferably less than 0.05 mg/ml, preferably less than 0.04 mg/ml, preferably less than 0.03 mg/ml, preferably 0.02 mg/ml, preferably less than 1µg/ml, preferably less than 100ng/ml, preferably less than 10ng/ml, more preferably still, less than 1ng/ml.

According to one embodiment of the invention, the agent that binds C5 is not an anti-C5 monoclonal antibody.

The invention also provides a therapeutically or prophylactically effective amount of an agent that inhibits eicosanoid activity for treating or preventing the inflammatory effects of viral infection of the respiratory tract ,wherein the agent is (i) a protein comprising or consisting of amino acids 19 to 168 or amino acids 1 to 168 of the amino acid sequence SEQ ID NO:2; (ii) a homologue of a protein as defined in (i) comprising or consisting of a sequence with at least 90% identity to the sequence of amino acids 19 to 168 or amino acids 1 to 168 of the amino acid sequence SEQ ID NO:2; or (iii) a fragment of a protein as defined in (i) above or of a homologue as defined in (ii) above wherein the fragment binds complement C5 and/or LTB4. The agent, as defined in the claims, according to this aspect of the invention may inhibit leukotrine B4 (LTB4) activity. In particular, the agent according to this aspect of the invention may bind LTB4. The ability of an agent to bind LTB4 may be determined by standard *in vitro* assays known in the art, for example by western blotting following incubation of the protein on the gel with labelled LTB4. The agent for use according to the invention may bind LTB4 with an IC₅₀ of less than 0.2 mg/ml, preferably less than 0.1 mg/ml, preferably less than 0.05 mg/ml, preferably less than 0.04 mg/ml, preferably less than 0.03 mg/ml, preferably 0.02 mg/ml, preferably less than 1µg/ml, preferably less than 100ng/ml, preferably less than 10ng/ml, more preferably still, less than 1ng/ml

The invention provides a therapeutically or prophylactically effective amount of an agent that inhibits:
a) the classical complement pathway, the alternative complement pathway and the lectin complement pathway; and
b) eicosanoid activity,
for use in treating or preventing the inflammatory effects of viral infection of the respiratory tract, wherein the agent is (i) a protein comprising or consisting of amino acids 19 to 168 or amino acids 1 to 168 of the amino acid sequence SEQ ID NO:2; (ii) a homologue of a protein as defined in (i) comprising or consisting of a sequence with at least 90% identity to the sequence of amino acids 19 to 168 or amino acids 1 to 168 of the amino acid sequence SEQ ID NO:2; or (iii) a fragment of a protein as defined in (i) above or of a homologue as defined in (ii) above wherein the fragment binds complement C5 and/or LTB4.

According to one embodiment of this aspect of the invention, the agent, as defined in the claims, binds both C5 and LTB4. The agent according to this embodiment may thus act to prevent the cleavage of complement C5 by C5 convertase into complement C5a and complement C5b-9 (MAC), and also to inhibit LTB4 activity.

The agent for use according to the invention described herein may be used to treat or prevent the inflammatory effects of viral infection of the upper or lower respiratory tracts. In particular, the methods and uses of the invention described herein may be used to treat or prevent respiratory failure caused by viral infection, including acute lung injury or acute respiratory distress syndrome. The agent for use according to the invention may also be used to treat or prevent the sequelae of respiratory failure caused by viral infection, including multi-organ failure.

The inflammation may be caused by any viral infection of the upper or lower respiratory tracts. In particular, the agent for use according to the invention may treat or prevent inflammatory effects caused by infection by pandemic influenza virus, such as influenza A H5N1 (avian influenza) and influenza A H1N1 (swine 'flu). The agent for use according to the invention may also be used to treat or prevent inflammatory effects caused by infection with SARS coronavirus.

Preferably, the agent for use according to the invention is derived from a haematophagous arthropod. The term "haematophagous arthropod" includes all arthropods that take a blood meal from a suitable host, such as insects, ticks, lice, fleas and mites. Preferably, the agent is derived from a tick, preferably from the tick *Ornithodoros moubata.*

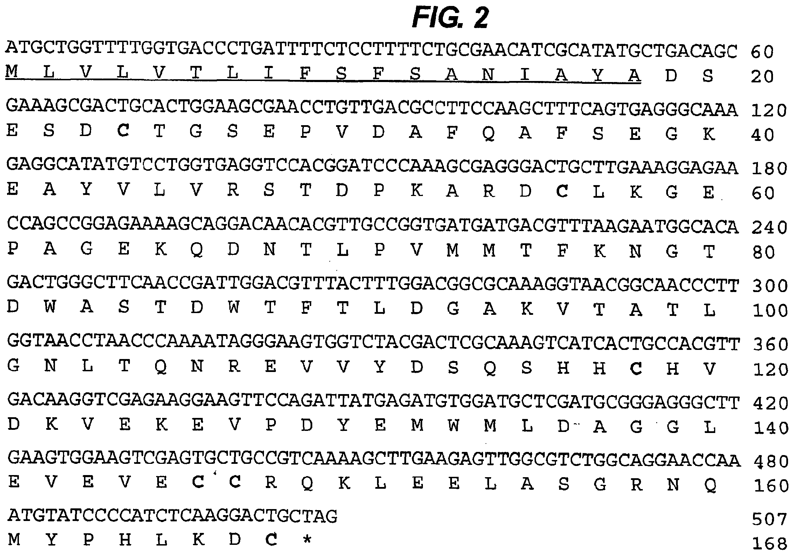

According to one embodiment of the invention, the agent is a protein comprising amino acids 19 to 168 of the amino acid sequence in Figure 2 or is a functional equivalent of this protein. The agent may be a protein consisting of amino acids 19 to 168 of the amino acid sequence in Figure 2 or be a functional equivalent of this protein.

According to an alternative embodiment, the protein for use according to this embodiment of the invention may comprise or consist of amino acids 1 to 168 of the amino acid sequence in Figure 2, or be a functional equivalent thereof. The first 18 amino acids of the protein sequence given in Figure 2 form a signal sequence which is not required for C5 binding or for LTB4 binding activity and so this may optionally be dispensed with, for example, for efficiency of recombinant protein production.

The protein having the amino acid sequence given in Figure 2, also referred to herein as the EV576 protein, was isolated from the salivary glands of the tick *Ornithodoros moubata.* EV576 is an outlying member of the lipocalin family and is the first lipocalin family member shown to inhibit complement activation. The EV576 protein inhibits the alternative, classical and lectin complement pathways by binding C5 and preventing its cleavage by C5 convertase into Complement C5a and Complement C5b - 9, thus inhibiting both the action of C5a peptide and the MAC. The EV576 protein also binds LTB4. The term "EV576 protein", as used herein, refers to the sequence given in Figure 2 with or without the signal sequence.

The EV576 protein and the ability of this protein to inhibit complement activation has been disclosed in [14], where the EV576 protein was referred to as the "OmCI protein". The EV576 protein has also been shown to be effective in the treatment of myasthenia gravis [15], respiratory disorders [16] and peripheral nerve disorders [17]. The ability of the EV576 protein to bind eicosanoids including LTB4 and its use in the treatment of diseases mediated by a leukotriene or hydroxyeicosanoid has been suggested in [18]. None of these disclosures suggest that the EV576 protein could be useful in the treatment or prevention of viral infection and in particular in the treatment or prevention of the inflammatory effects of viral infection of the respiratory tract.

It has now been found that the EV576 protein is surprisingly effective in the treatment and prevention of the inflammatory effects of viral infection of the respiratory tract. The data presented herein demonstrate that, in a murine model of human H1N1 influenza infection of the respiratory tract, mice treated with EV576 had significantly lower levels of protein and total cells, lower levels of complement C5a, lower levels of inflammatory cytokines IL-6, IL-1β, and CXCL2, and highly significantly lower neutrophils compared with vehicle-treated mice. EV576 thus represents a potential human therapy for the treatment and prevention of the inflammatory effects of viral infection of the respiratory tract. The surprising effectiveness of EV576 in the treatment of respiratory disorders may be due to the fact that it acts by binding C5, thus inhibiting the formation of C5a and MAC, or due to its LTB4 binding activity.

According to a further embodiment of the invention, the agent may be a nucleic acid molecule encoding the EV576 protein or a functional equivalent thereof. For example, gene therapy may be employed to effect the endogenous production of the EV576 protein by the relevant cells in the subject, either *in vivo* or *ex vivo.* Another approach is the administration of "naked DNA" in which the therapeutic gene is directly injected into the bloodstream or into muscle tissue.

Preferably, such a nucleic acid molecule comprises or consists of bases 55 to 507 of the nucleotide sequence in Figure 2. This nucleotide sequence encodes the EV576 protein in Figure 2 without the signal sequence. The first 54 bases of the nucleotide sequence in Figure 2 encode the signal sequence which is not required for complement inhibitory activity or LTB4 binding activity. Alternatively, the nucleic acid molecule may comprise or consist of bases 1 to 507 of the nucleic acid sequence in Figure 2, which encodes the protein with the signal sequence.

The EV576 protein has been demonstrated to bind to C5 and prevent its cleavage by C5 convertase in rat, mouse and human serum with an IC₅₀ of approximately 0.02mg/ml. Preferably, functional equivalents of the EV576 protein which retain the ability to bind C5 with an IC₅₀ of less than 0.2 mg/ml, preferably less than 0.1 mg/ml, preferably less than 0.05 mg/ml, preferably less than 0.02 mg/ml, preferably less than 1µg/ml, preferably less than 100ng/ml, preferably less than 10ng/ml, more preferably still, less than 1ng/ml.

The EV576 protein has also been demonstrated to bind LTB4. Functional equivalents of the EV576 protein may also retain the ability to bind LTB4 with a similar affinity as the EV576 protein.

In one respect, the term "functional equivalent" is used herein to describe homologues and fragments of the EV576 protein which: a) retain its ability to bind C5, and to prevent the cleavage of complement C5 by C5 convertase into complement C5a and complement C5b-9; and/or b) retain its ability to bind LTB4..

The term "functional equivalent" also refers to molecules that are structurally similar to the EV576 protein or that contain similar or identical tertiary structure, particularly in the environment of the active site or active sites of the EV576 protein that binds to C5 and/or LTB4, such as synthetic molecules. Amino acids in EV576 that are likely to be required for LTB4 binding are described in [18].

The term "homologue" is meant to include reference to paralogues and orthologues of the EV576 sequence that is explicitly identified in Figure 2, including, for example, the EV576 protein sequence from other tick species, including *Rhipicephalus appendiculatus, R. sanguineus, R. bursa, A. americanum, A. cajennense, A. hebraeum, Boophilus microplus, B. annulatus, B. decoloratus, Dermacentor reticulatus, D. andersoni, D. marginatus, D. variabilis, Haemaphysalis inermis, Ha. leachii, Ha. punctata, Hyalomma anatolicum anatolicum, Hy. dromedarii, Hy. marginatum marginatum, Ixodes ricinus, I. persulcatus, I. scapularis, I. hexagonus, Argas persicus, A. reflexus, Ornithodoros erraticus, O. moubata moubata, O. m. porcinus, and O. savignyi.* The term "homologue" is also meant to include the equivalent EV576 protein sequence from mosquito species, including those of the *Culex, Anopheles* and *Aedes* genera, particularly *Culex quinquefasciatus, Aedes aegypti* and *Anopheles gambiae;* flea species, such as *Ctenocephalides felis* (the cat flea); horseflies; sandflies; blackflies; tsetse flies; lice; mites; leeches; and flatworms. The native EV576 protein is thought to exist in *O. moubata* in another three forms of around 18kDa and the term "homologue" is meant to include these alternative forms of EV576.

Methods for the identification of homologues of the EV576 sequence given in Figure 2 will be clear to those of skill in the art. For example, homologues may be identified by homology searching of sequence databases, both public and private. Conveniently, publicly available databases may be used, although private or commercially-available databases will be equally useful, particularly if they contain data not represented in the public databases. Primary databases are the sites of primary nucleotide or amino acid sequence data deposit and may be publicly or commercially available. Examples of publicly-available primary databases include the GenBank database (http://www.ncbi.nlm.nih.gov/), the EMBL database (http://www.ebi.ac.uk/), the DDBJ database (http://www.ddbj.nig.ac.jp/), the SWISS-PROT protein database (http://expasy.hcuge.ch/), PIR (http://pir.georgetown.edu/), TrEMBL (http://www.ebi.ac.uk/), the TIGR databases (see http://www.tigr.org/tdb/index.html), the NRL-3D database (http://www.nbrfa.georgetown.edu), the Protein Data Base (http://www.rcsb.org/pdb), the NRDB database (ftp://ncbi.nlm.nih.gov/pub/nrdb/README), the OWL database (http://www.biochem.ucl.ac.uk/bsm/dbbrowser/OWL/) and the secondary databases PROSITE (http://expasy.hcuge.ch/sprot/prosite.html), PRINTS (http://iupab.leeds.ac.uk/bmb5dp/prints.html), Profiles (http://ulrec3.unil.ch/software/PFSCAN_form.html), Pfam (http://www.sanger.ac.uk/software/pfam), Identify (http://dna.stanford.edu/identify/) and Blocks (http://www.blocks.fhcrc.org) databases. Examples of commercially-available databases or private databases include PathoGenome (Genome Therapeutics Inc.) and PathoSeq (previously of Incyte Pharmaceuticals Inc.).

Typically, greater than 30% identity between two polypeptides (preferably, over a specified region such as the active site) is considered to be an indication of functional equivalence and thus an indication that two proteins are homologous. Preferably, proteins that are homologues have a degree of sequence identity with the EV576 protein sequence identified in Figure 2 of greater than 60%. More preferred homologues have degrees of identity of greater than 90% with the EV576 protein sequence given in Figure 2. Percentage identity, as referred to herein, is as determined using BLAST version 2.1.3 using the default parameters specified by the NCBI (the National Center for Biotechnology Information; http://www.ncbi.nlm.nih.gov/) [Blosum 62 matrix; gap open penalty=11 and gap extension penalty=1].

Homologues of the EV576 protein sequence given in Figure 2 include mutants containing amino acid substitutions, insertions or deletions from the wild type sequence, for example, of 1, 2, 3, 4, 5, 7, 10 or more amino acids, provided that such mutants retain the ability to bind C5. Mutants thus include proteins containing conservative amino acid substitutions that do not affect the function or activity of the protein in an adverse manner. This term is also intended to include natural biological variants (e.g. allelic variants or geographical variations within the species from which the EV576 proteins are derived). Mutants with improved ability to bind C5 and/or LTB4 may also be designed through the systematic or directed mutation of specific residues in the protein sequence.

Fragments of the EV576 protein and of homologues of the EV576 protein are also embraced by the term "functional equivalents" providing that such fragments retain the ability to bind C5 and/or LTB4. Fragments may include, for example, polypeptides derived from the EV576 protein sequence which are less than 150 amino acids, less than 125 amino acids, less than 100 amino acids, less than 75 amino acids, less than 50 amino acids, or even 25 amino acids or less, provided that these fragments retain the ability to bind to complement C5.

Included as such fragments are not only fragments of the *O. moubata* EV576 protein that is explicitly identified herein in Figure 2, but also fragments of homologues of this protein, as described above. Such fragments of homologues will typically possess greater than 60% identity with fragments of the EV576 protein sequence in Figure 2, although more preferred fragments of homologues will display degrees of identity of greater than 90%, with fragments of the EV576 protein sequence in Figure 2. Fragments with improved may, of course, be rationally designed by the systematic mutation or fragmentation of the wild type sequence followed by appropriate activity assays. Fragments may exhibit similar or greater affinity for C5 and/or LTB4 as EV576.

A functional equivalent may be a fusion protein, obtained, for example, by cloning a polynucleotide encoding the EV576 protein in frame to the coding sequences for a heterologous protein sequence. The term "heterologous", when used herein, is intended to designate any polypeptide other than the EV576 protein or its functional equivalent. Example of heterologous sequences, that can be comprised in the soluble fusion proteins either at N- or at C-terminus, are the following: extracellular domains of membrane-bound protein, immunoglobulin constant regions (Fc region), multimerization domains, domains of extracellular proteins, signal sequences, export sequences, or sequences allowing purification by affinity chromatography. Many of these heterologous sequences are commercially available in expression plasmids since these sequences are commonly included in the fusion proteins in order to provide additional properties without significantly impairing the specific biological activity of the protein fused to them [19]. Examples of such additional properties are a longer lasting half-life in body fluids, the extracellular localization, or an easier purification procedure as allowed by a tag such as a histidine or HA tag.

The EV576 protein and functional equivalents thereof, may be prepared in recombinant form by expression in a host cell. Such expression methods are well known to those of skill in the art and are described in detail by [20] and [21]. Recombinant forms of the EV576 protein and functional equivalents thereof are preferably unglycosylated.

The proteins and fragments for use according to the present invention can also be prepared using conventional techniques of protein chemistry. For example, protein fragments may be prepared by chemical synthesis. Methods for the generation of fusion proteins are standard in the art and will be known to the skilled reader. For example, most general molecular biology, microbiology recombinant DNA technology and immunological techniques can be found in [20] or [22].

The subject to which the agent for use in accordance with the invention is administered is preferably a mammal, preferably a human. The subject to which the agent is administered may also be suffering from a viral infection of the upper or lower respiratory tract, such as pandemic influenza virus, including influenza A H5N1 (avian influenza) and influenza A H1N1 (swine 'flu), or SARS coronavirus.

The agent is administered in a therapeutically or prophylactically effective amount. The term "therapeutically effective amount" refers to the amount of agent needed to treat or ameliorate the inflammation associated with the viral infection The term "prophylactically effective amount" used herein refers to the amount of agent needed to prevent inflammation associated with the viral invention.

Preferably, the dose of the agent is sufficient to bind as much available C5 as possible in the subject, more preferably, all available C5. The dose of the agent may alternatively be sufficient to bind as much available LTB4 as possible in the subject, more preferably, all available LTB4. In some aspects, the dose of the agent is sufficient to binds as much available C5 and LTB4 as possible, for example all available C5 and LTB4. The dose of the agent supplied is at least twice the molar dose needed to bind all available C5 and/or LTB4 in the subject. The dose of the agent supplied may be 2.5 times, 3 times or 4 times the molar dose needed to bind all available C5 and/or LTB4 in the subject. Preferably, the dose is from 0.0001 mg/kg (mass of drug compared to mass of patient) to 20 mg/kg, preferably 0.001 mg/kg to 10 mg/kg, more preferably 0.2 mg/kg to 2 mg/kg.

The frequency with which the dose needs to be administered will depend on the half-life of the agent involved. Where the agent is the EV576 protein or a functional equivalent thereof, the dose may be administered as a continuous infusion, in bolus doses or on a daily basis, twice daily basis, or every two, three, four days, five, six, seven, 10, 15 or 20 days or more.

The exact dosage and the frequency of doses may also be dependent on the patient's status at the time of administration. Factors that may be taken into consideration when determining dosage include the severity of the disease state in the patient, the general health of the patient, the age, weight, gender, diet, time and frequency of administration, drug combinations, reaction sensitivities and the patient's tolerance or response to therapy. The precise amount can be determined by routine experimentation, but may ultimately lie with the judgement of the clinician.

The agent will generally be administered as part of a pharmaceutically acceptable carrier. The term "pharmaceutically acceptable carrier", as used herein, includes genes, polypeptides, antibodies, liposomes, polysaccharides, polylactic acids, polyglycolic acids and inactive virus particles or indeed any other agent provided that the carrier does not itself induce toxicity effects or cause the production of antibodies that are harmful to the individual receiving the pharmaceutical composition. Pharmaceutically acceptable carriers may additionally contain liquids such as water, saline, glycerol, ethanol or auxiliary substances such as wetting or emulsifying agents, pH buffering substances and the like. The pharmaceutical carrier employed will thus vary depending on the route of administration. Carriers may enable the pharmaceutical compositions to be formulated into tablets, pills, dragees, capsules, liquids, gels, syrups, slurries, suspensions to aid intake by the patient. A thorough discussion of pharmaceutically acceptable carriers is available in [23].

The agent may be delivered by any known route of administration. The agent may be delivered nasally, by inhalation, for example, using a metered-dose inhaler, nebuliser, dry powder inhaler, or nasal inhaler. The agent may be delivered by a parenteral route (*e.g.* by injection, either subcutaneously, intraperitoneally, intravenously or intramuscularly or delivered to the interstitial space of a tissue). The compositions can also be administered into a lesion. Other modes of administration include oral and pulmonary administration, suppositories, and transdermal or transcutaneous applications, needles, and hyposprays. The agent may be administered alone or as part of a treatment regimen also involving the administration of other drugs currently used in the treatment of patients with respiratory disorders. For example, the agent may be administered in combination with the infusion of additional anti-viral drugs and/or oxygen treatment.

The agent may be administered simultaneously, sequentially or separately with the other drug(s). For example, the agent may be administered before or after administration of the other drug(s).

The invention thus provides an agent that binds C5 and/or LTB4, preferably the EV576 protein or a functional equivalent thereof, for treating or preventing the inflammatory effects of viral infection of the respiratory tract in a subject, wherein said subject has been pre-treated with an anti-viral drug. Examples of anti-viral drugs that may be used in this aspect of the invention include zanamivir (Relenza) and oseltamivir (Tamiflu).

Various aspects and embodiments of the present invention will now be described in more detail by way of example. It will be appreciated that modification of detail may be made without departing from the scope of the invention.

### Brief description of Figures:

**Figure 1****:** Schematic diagram of classical and alternative pathways of complement activation. Enzymatic components, dark grey. Anaphylatoxins enclosed in starbursts.
**Figure 2****:** Primary sequence of EV576. Signal sequence underlined. Cysteine residues in bold type. Nucleotide and amino acid number indicated at right.
**Figure 3****:** Reduction in neutrophils at 10⁴ plaque forming units (PFU) following administration of EV576 to a murine model of swine flu infection compared with administration of EV131 or a vehicle in the same model.
**Figure 4****:** Reduction in total cells at 10⁴ plaque forming units (PFU) following administration of EV576 to a murine model of swine flu infection compared with administration of EV131 or a vehicle in the same model.
**Figure 5****:** Reduction in total protein at 10⁴ plaque forming units (PFU) following administration of EV576 to a murine model of swine flu infection compared with administration of EV131 or a vehicle in the same model.
**Figure 6****:** Reduction in neutrophils at 10⁶ plaque forming units (PFU) following administration of EV576 to a murine model of swine flu infection compared with a vehicle in the same model.
**Figure 7****:** Reduction in total cells at 10⁶ plaque forming units (PFU) following administration of EV576 to a murine model of swine flu infection compared with a vehicle in the same model.
**Figure 8****:** Reduction in total protein at 10⁶ plaque forming units (PFU) following administration of EV576 to a murine model of swine flu infection compared with a vehicle in the same model.
**Figure 9****:** Reduction in IL-6 level at 10⁴ plaque forming units (PFU) following administration of EV576 (OmCI) to a murine model of swine flu infection compared with administration of EV131 or a vehicle in the same model.
**Figure 10****:** Reduction in CXC2 level at 10⁴ plaque forming units (PFU) following administration of EV576 (OmCI) to a murine model of swine flu infection compared with administration of EV131 or a vehicle in the same model.
**Figure 11****:** Reduction in IL-1β at 10⁴ plaque forming units (PFU) following administration of EV576 (OmCI) to a murine model of swine flu infection compared with administration of EV131 or a vehicle in the same model.
**Figure 12****:** Reduction in IL-6 level at 10⁶ plaque forming units (PFU) following administration of EV576 (OmCI) to a murine model of swine flu infection compared with a vehicle in the same model.
**Figure 13****:** Reduction in CXC2 level at 10⁶ plaque forming units (PFU) following administration of EV576 (OmCI) to a murine model of swine flu infection compared with a vehicle in the same model.
**Figure 14****:** Reduction in IL-1β at 10⁶ plaque forming units (PFU) following administration of EV576 (OmCI) to a murine model of swine flu infection compared with a vehicle in the same model.
**Figure 15****:** Relative reduction in neutrophils per 100mg lung tissue at 10⁴ plaque forming units (PFU) following administration of EV576 to a murine model of swine flu infection compared with administration of EV131 or a vehicle in the same model.
**Figure 16****:** Relative reduction in neutrophils per 100mg lung tissue at 10⁶ plaque forming units (PFU) following administration of EV576 to a murine model of swine flu infection compared with administration of EV131 or a vehicle in the same model.
**Figure 17****:** Overall histopathological score at 10⁴ plaque forming units (PFU) following administration of EV576 to a murine model of swine flu infection compared with administration of EV131 or a vehicle in the same model.
**Figure 18****:** Overall histopathological score at 10⁶ plaque forming units (PFU) following administration of EV576 to a murine model of swine flu infection compared with a vehicle in the same model.
**Figure 19****:** C5a level in Bronchoalveolar lavage fluid (BALF) in a murine model of swine flu infection at an inoculum level of 10⁴ plaque forming units (PFU) compared with mock inoculation.
**Figure 20****:** C5a level in Bronchoalveolar lavage fluid (BALF) in a murine model of swine flu infection at an inoculum level of 10⁶ plaque forming units (PFU) compared with mock inoculation.
**Figure 21****:** Reduction in C5a level at 10⁴ plaque forming units (PFU) following administration of EV576 to a murine model of swine flu infection compared with administration of EV131 or a vehicle in the same model.
**Figure 22****:** Reduction in C5a level at 10⁶ plaque forming units (PFU) following administration of EV576 to a murine model of swine flu infection compared with administration of a vehicle in the same model.

### Example 1

EV576 protein having the amino acid sequence shown in Figure 2 was tested in a murine model of human H1N1 influenza infection of the respiratory tract.

### Methods

BalbC mice were infected transnasally with either a sub-lethal dose (10⁴ PFU) or a lethal dose (10⁶ PFU) of human H1N1 virus obtained from the *Institut Pasteur* or sham infected using phosphate buffered saline (PBS).

Signs of lower respiratory tract infection (weight loss and respiratory congestion) developed within 4 days in low inoculum animals and more rapidly in the high inoculum group who all succumbed to the virus by the third day.

EV576 protein having the amino acid sequence shown in Figure 2 was given by intraperitoneal injection 250µg 30 minutes pre-infection and on Day 1, 200µg on days 2, 3 and 4 and 170µg on Day 5. rEV131, a similarly sized lipocalin to EV576 but which is known not to inhibit the complement system, and PBS were used as controls.

High inoculum mice received only the first two doses as they were all dead by Day 3. Bronchoalveolar lavage (BAL) was performed on Day 3 for high inoculum mice. Bronchoalveolar lavage (BAL) was performed on Day 6 for low inoculum mice, 24 hours after the last dosing and immediately prior to sacrifice. Lavage fluid was assayed for total cells, neutrophils, total protein and cytokines (IL-1β, IL-6 and CXCL2).

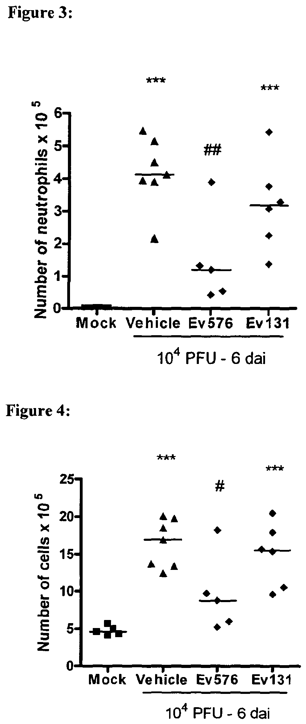

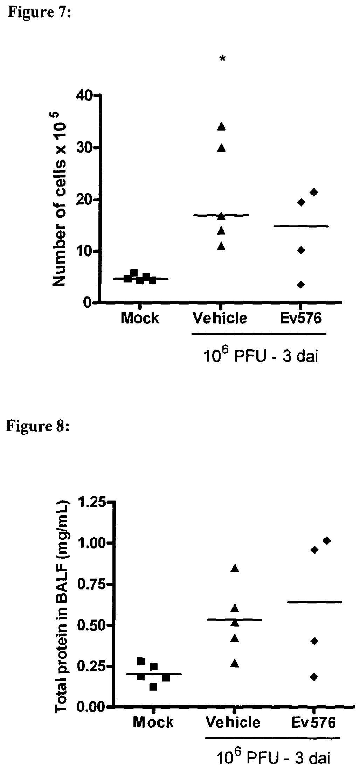

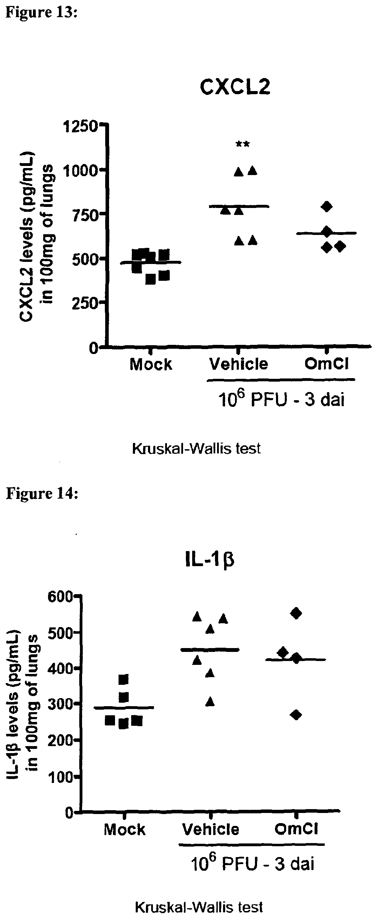

The results are shown in Figures 3-16.

All parameters were found to be highly significantly raised in vehicle treated mice compared to mock (sham) treated mice.

### Results

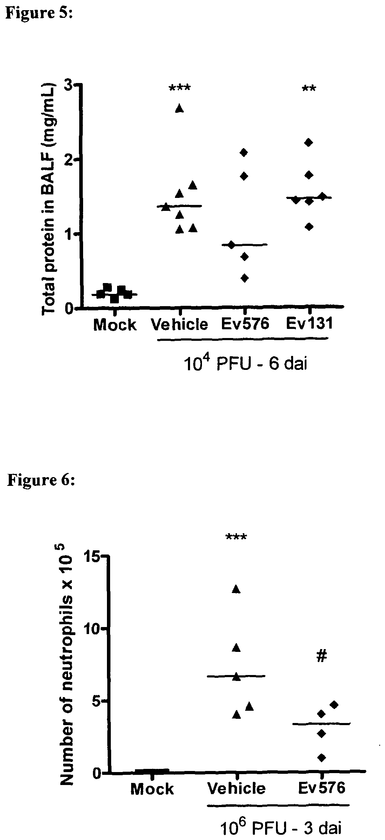

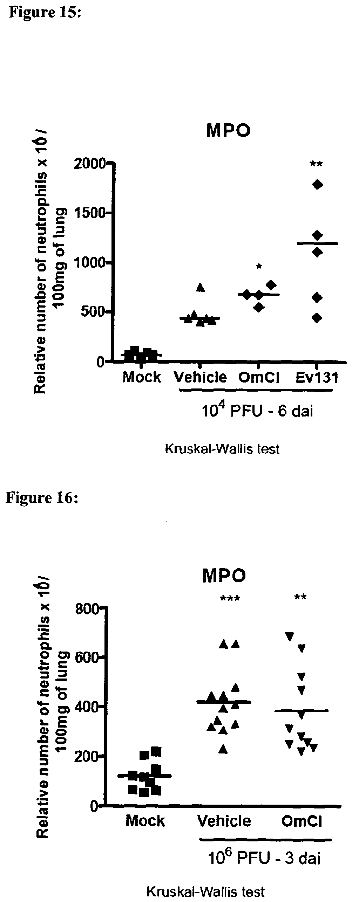

Mice treated with EV576 had significantly lower levels of protein (Figure 5) and total cells (Figure 4), and highly significantly lower neutrophils (Figure 3 and 15) compared with vehicle treated mice at 10⁴ plaque forming units (PFU). Mice treated with EV576 also had significantly lower neutrophils at 10⁶ PFU compared to vehicle.

At 10⁴ PFU vehicle treated and rEV131 mice had highly significantly (p<0.001) raised total cells, neutrophils and protein compared to sham (mock) treated animals. Animals treated with EV576 had significantly less elevation of total cells (p<0.05) and neutrophils (p<0.01) than vehicle treated ones (Figures 3 and 4). At 10⁶ PFU only total cells (p<0.05) and neutrophils (p<0.001) were elevated compared to sham treated animals and neutrophil elevation was significantly less (p<0.05) in mice treated with EV576 compared to vehicle (Figures 6-8 and 16) but since BAL was performed on moribund animals at Day 3 instead of Day 6 it is likely that the effects of the respiratory complications of H1N1 influenza had not had time to develop fully and therefore any possible protective effects of complement inhibition were not as apparent.

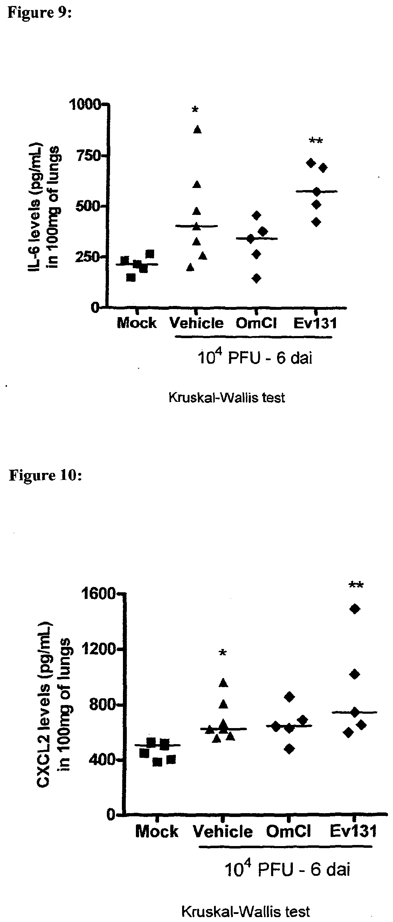

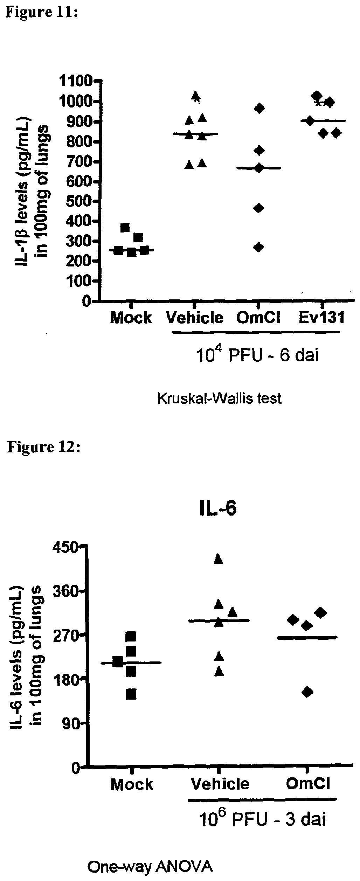

In the low inoculum group levels of the inflammatory cytokines IL-1β, IL-6 and CXCL2 in BAL fluid were found to be significantly (p<0.05) or highly significantly (p<0.01) elevated in vehicle and rEV131 treated groups compared to sham (mock) treated animals (Figures 9-11) but, although elevated, were not significantly different in EV576 treated animals. As with the cellular results the differences were less marked in the high inoculum group (Figures 12-14).

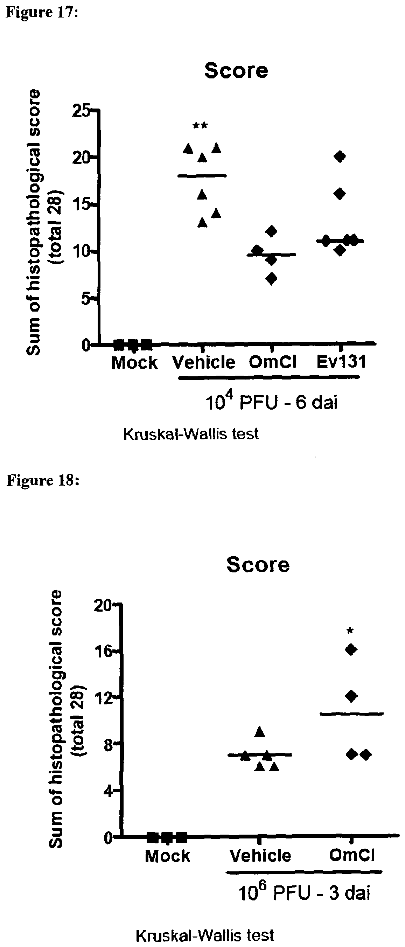

The sum of all the histopathological scores of lung inflammation is show in Figures 17 and 18 for low and high inoculum mice respectively. The sum of the histopathological scores was calculated using scores for airway inflammation, vascular inflammation, parenchymal inflammation, neutrophilic inflammation and epithelial injury. EV576 reduced epithelial damage and inflammation in the low inoculum groups. The histamine scavenging protein EV131 was less effective.

These data demonstrate that EV576 significantly inhibits the inflammatory effects of H1N1 infection. EV576 is known to inhibit the complement system via binding of C5 and to inhibit eicosanoid activity. These data suggest that EV576 and other agents having similar C5 binding properties may be effective in reducing the lung inflammatory effects of viral infection.

### Example 2

EV576 protein having the amino acid sequence shown in Figure 2 was tested in a murine model of human H1N1 influenza infection of the respiratory tract for its effect on complement C5a level.

### Methods

BalbC mice were infected and treated as described above in Example 1.

C5a levels in BALF were measured in mock inoculated animals on day, on days 1, 4, 7, and 10 in animals exposed to a sub-lethal dose (10⁴ PFU), and on days 1, 3 and 5 in animals exposed to a lethal dose (10⁶ PFU) of human H1N1 virus.

On day 6, C5a levels in BALF were measured in low inoculum animals that had also been treated with vehicle, EV131 or EV576. On day 3, C5a levels in BALF were measured in high inoculum animals that had also been treated with vehicle or EV576.

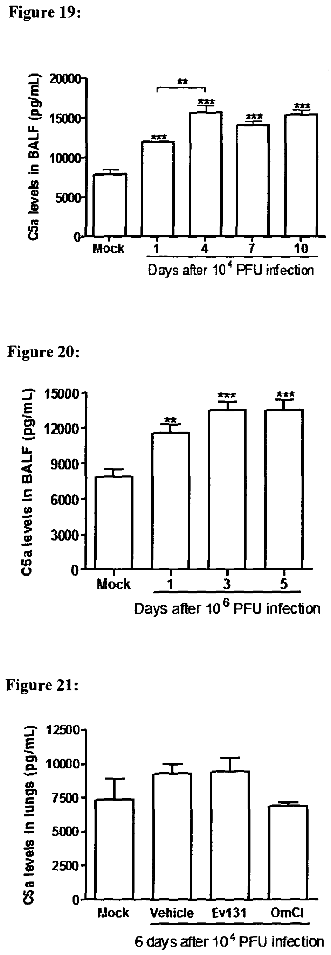

Results are shown in Figures 19 to 22.

### Results

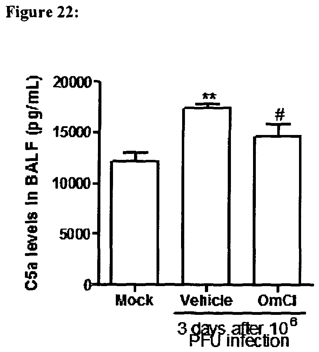

There is a significant rise in C5a in BAL fluid for up to 10 days following infection at both the high and low inoculum levels (Figures 19 and 20). Mice treated with EV576 had a significantly attenuated rise in C5a level. Vehicle and EV131 did not attenuate the rise in C5a level (Figures 21 and 22).

This is the first time that a direct role for complement has demonstrated in inflammation caused by H1N1 influenza infection. This rise in complement can be blocked using the complement inhibitor EV576. The ability of EV576 to block complement and associated inflammation suggests that it will be effective in the treatment of H1N1 influenza infection.

### Example 3

The cytokine storm and associated inflammation that follows H1N1 infection is also present following H5N1 infection. The ability of EV576 to block complement rise and associated inflammation following H1N1 infection is therefore expected to be replicated in following H5N1 infection. EV576 protein having the amino acid sequence shown in Figure 2 can be tested in a murine model of human H5N1 influenza infection of the respiratory tract using the experimental protocol reported above and in reference 24. The effect of oseltamivir (Tamiflu) in combination with EV576 following infection with H5N1 or H1N1 influenza can also be determined.

### Methods

The experimental protocol is similar to that reported in reference 24.

Pathogen-free, female, 8 week old mice with a standardised body weight of 20-23g (e.g. FVB/J strain) are infected transnasally with human H5N1 virus or H1N1 virus obtained from the *Institut Pasteur* or sham infected using phosphate buffered saline (PBS).

EV576 protein having the amino acid sequence shown in Figure 2 is given by intraperitoneal injection. Mice may also receive oseltamivir. PBS is used as a control. Four groups may be enrolled in each infection model : (1) virus+PBS, (2) virus+EV576, (3) virus+EV576+oseltamivir and (4) virus+oseltamivir. The infection and treatment schedule is shown in the table below:

| Experimental campaign | Group | Timepoints | | | | | |
|---|---|---|---|---|---|---|---|
| | | Day 2 pi | Day 3 pi | Day 4 pi | Day 6 pi | Day 7 pi | Day 8 pi |
| | | | | | | | |
| H5N1 | Virus+PBS+PBS | 5 | 5 | 5 | | | |
| | Virus+CVS+PBS | 5 | 5 | 5 | | | |
| | Virus+CVS+OSTMV | 5 | 5 | 5 | | | |
| | Virus+PBS+OSTMV | 5 | 5 | 5 | | | |
| | | | | | | | |
| H1N1 | Virus+PBS+PBS | 5 | | 5 | 5 | 5 | 5 |
| | Virus+CVS+PBS | 5 | | 5 | 5 | 5 | 5 |
| | Virus+CVS+OSTMV | 5 | | 5 | 5 | 5 | 5 |
| | Virus+PBS+OSTMV | 5 | | 5 | 5 | 5 | 5 |
| | | | | | | | |
| Total mice | | **40** | **20** | **40** | **20** | **20** | **20** |
| | | | | | | | |

| | | | | | | | |
|---|---|---|---|---|---|---|---|
| CVS stands for coversin (EV576) and OSTMV for oseltamivir. Numbers may be doubled. | | | | | | | |

The following signs of infection and readouts of treatment effects can be measured:
- Bodyweight, daily
- Group-specific % survival
- Photo of each lung, dorso-ventral view, each timepoint
- Lung weight, each timepoint
- Lung bulk virus titer, each timepoint
- Lung bulk myeloperoxidase activity, each timepoint
- Option 1 : histopathologic exam., each timepoint
- Option 2 : lung bulk neutrophil chemokines (KC and MIP-2)
- Option 3 : lung bulk monocyte/NK/T cells chemokines (MCP1, MIP1α)
- Option 4 : lung bulk cytokines (IL-6, TNFα, IFNα, IFNγ, IL-1β)
- Option 5 : global virus distribution in lungs, by IF
- Option 6 : Cell countings in BALF by flow cytometry
- Time points for collection of readouts will be day 2, day 3 and day 4 post infection after H5N1 or day 2, day 4, day 6, day 7, day 8 post infection after H1N1. Pre-infection values will also be determined.

### References

[1] Mastellos, D., et al., Clin Immunol, 2005. 115(3): p. 225-35
[2] Peiris JSM, Cheung CY, Leung CYH, Nicholls JM. Innate immune responses to influenza A H5N1 :friend or foe? Trends in Immunology 2009; 30,12: 574-584.
[3] Lee SMY, Gardy JL, Cheung CY et al. Systems-level comparison of host-responses elicited by avian H5N1 and seasonal H1N1 influenza viruses in primary human macrophages. PLoS One, December 2009; 4, 12: 1-11.
[4] Guo, R.F. and P.A. Ward, Annu Rev Immunol, 2005, 23: p. 821-52
[5] Neumann, E., et al., Arthritis Rheum, 2002. 46(4): p. 934-45
[6] Williams, A.S., et al., Arthritis Rheum, 2004, 50(9): p. 3035-44
[7] Quigg, R.J., Curr Dir Autoimmun, 2004. 7: p. 165-80
[8] Papagianni, A.A., et al., Nephrol Dial Transplant, 2002, 17(1): p. 57-63
[9] He, C., et al., J Immunol, 2005. 174(9): p. 5750-7
[10] Mead, R.J., et al., J Immunol, 2002. 168(1): p. 458-65
[11] Nakashima, S., et al., J Immunol, 2002. 169(8): p. 4620-7
[12] Mizuno, M. and D.S. Cole, Expert Opin Investig Drugs, 2005. 14(7): p. 807-21
[13] Allegretti, M., et al.,. Curr Med Chem, 2005. 12(2): p. 217-36
[14] WO2004/106369
[15] WO/2007/028968
[16] WO/2008/029169
[17] WO/2008/029167
[18] WO2009/098454
[19] Terpe K, Appl Microbiol Biotechnol, 60: 523-33, 2003
[20] Sambrook *et al* (2000)
[21] Fernandez & Hoeffler (1998)
[22] Ausubel *et al.* (1991)
[23] Remington's Pharmaceutical Sciences; Mack Pub. Co., N.J. 1991
[24] Garigliany et al., (2010) Emerg Infect Dis 16:595-603

## Claims

1. A therapeutically or prophylactically effective amount of an agent that:
a) inhibits the classical complement pathway, the alternative complement pathway and the lectin complement pathway; and/or
b) inhibits eicosanoid activity
for use in treating or preventing the inflammatory effects of viral infection of the respiratory tract;
wherein the agent is:
(i) a protein comprising or consisting of amino acids 19 to 168 or amino acids 1 to 168 of the amino acid sequence SEQ ID NO:2;
(ii) a homologue of a protein as defined in (i) comprising or consisting of a sequence with at least 90% identity to the sequence of amino acids 19 to 168 or amino acids 1 to 168 of the amino acid sequence SEQ ID NO:2; or
(iii) a fragment of a protein as defined in (i) above or of a homologue as defined in (ii) above, wherein the fragment binds complement C5 and/or LTB4.

2. An agent for use according to claim 1, wherein the agent binds complement C5.

3. An agent for use according to claim 1, wherein the agent inhibits the cleavage of complement C5 by C5 convertase into complement C5a and complement C5b-9 (MAC).

4. An agent for use according to any one of claims 1 to 3, wherein the agent binds LTB4.

5. An agent for use according to any one of claims 1 to 4 wherein the agent is derived from a haematophagous arthropod.

6. An agent for use according to any one of claims 1 to 5 wherein the agent is administered as a nucleic acid molecule encoding a protein, homologue or fragment as recited in claim 1.

7. An agent for use according to claim 6 wherein the nucleic acid molecule comprises or consists of bases 55 to 507 of the nucleotide sequence SEQ ID NO:1.

8. An agent for use according to claim 7 wherein the nucleic acid molecule comprises or consists of bases 1 to 507 of the nucleotide sequence SEQ ID NO:1.

9. An agent for use according to any one of claims 1 to 8 wherein the subject is a mammal, preferably a human.

10. An agent for use according to any one of claims 4 to 9 wherein the agent is administered in a dose sufficient to bind as much available C5 and/or LTB4 as possible in the subject, more preferably, all available C5.

11. An agent for use according to any one of claims 1 to 10 wherein the inflammatory effect of viral infection of the respiratory tract is respiratory failure, such as acute lung injury or acute respiratory distress syndrome, or the sequelae of respiratory failure, including multi-organ failure.

12. An agent for use according to any one of claims 1 to 11, wherein the viral infection is an infection of the upper or lower respiratory tracts, including pandemic influenza virus, such as influenza A H5N1 (avian influenza) and influenza A H1N1 (swine 'flu), and SARS coronavirus.

## Patentansprüche

1. Therapeutisch oder prophylaktisch wirksame Menge eines Mittels, das:
a) den klassischen Komplementweg, den alternativen Komplementweg und den Lectin-Komplementweg hemmt; und/oder
b) die Eicosanoidaktivität hemmt
zur Verwendung bei der Behandlung oder Prävention der Entzündungswirkungen einer Virusinfektion der Atemwege;
wobei das Mittel:
(i) ein Protein, das die Aminosäuren 19 bis 168 oder die Aminosäuren 1 bis 168 der Aminosäuresequenz SEQ ID NO: 2 umfasst oder daraus besteht;
(ii) ein Homologon eines Proteins wie in (i) definiert, das eine Sequenz mit mindestens 90% Identität zu der Sequenz der Aminosäuren 19 bis 168 oder der Aminosäuren 1 bis 168 der Aminosäuresequenz SEQ ID NO: 2 umfasst oder daraus besteht; oder
(iii) ein Fragment eines Proteins wie in (i) oben definiert oder eines Homologons wie in (ii) oben definiert, wobei das Fragment Komplement C5 und/oder LTB4 bindet,
ist.

2. Mittel zur Verwendung nach Anspruch 1, wobei das Mittel Komplement C5 bindet.

3. Mittel zur Verwendung nach Anspruch 1, wobei das Mittel die Spaltung von Komplement C5 durch C5-Konvertase zu Komplement C5a und Komplement C5b-9 (MAC) hemmt.

4. Mittel zur Verwendung nach einem der Ansprüche 1 bis 3, wobei das Mittel LTB4 bindet.

5. Mittel zur Verwendung nach einem der Ansprüche 1 bis 4, wobei das Mittel von einem hämatophagen Arthropoden stammt.

6. Mittel zur Verwendung nach einem der Ansprüche 1 bis 5, wobei das Mittel als Nukleinsäuremolekül verabreicht wird, das ein Protein, Homologon oder Fragment nach Anspruch 1 codiert.

7. Mittel zur Verwendung nach Anspruch 6, wobei das Nukleinsäuremolekül die Basen 55 bis 507 der Nukleotidsequenz SEQ ID NO: 1 umfasst oder daraus besteht.

8. Mittel zur Verwendung nach Anspruch 7, wobei das Nukleinsäuremolekül die Basen 1 bis 507 der Nukleotidsequenz SEQ ID NO: 1 umfasst oder daraus besteht.

9. Mittel zur Verwendung nach einem der Ansprüche 1 bis 8, wobei das Individuum ein Säugetier, vorzugsweise ein Mensch, ist.

10. Mittel zur Verwendung nach einem der Ansprüche 4 bis 9, wobei das Mittel in einer Dosis verabreicht wird, die ausreicht, dass es so viel verfügbares C5 und/oder LTB4 wie möglich in dem Individuum, stärker bevorzugt sämtliches verfügbares C5, bindet.

11. Mittel zur Verwendung nach einem der Ansprüche 1 bis 10, wobei die Entzündungswirkung der Virusinfektion der Atemwege ein Atemversagen, wie eine akute Lungenverletzung oder ein akutes Atemnotsyndrom oder die Folgen eines Atemversagens, einschließlich Multiorganversagen, ist.

12. Mittel zur Verwendung nach einem der Ansprüche 1 bis 11, wobei die Virusinfektion eine Infektion der oberen oder unteren Atemwege ist, einschließlich des pandemischen Influenzavirus wie Influenza A H5N1 (Vogelgrippe) und Influenza A H1N1 (Schweinegrippe) und SARS-Coronavirus.

## Revendications

1. Quantité, efficace sur le plan thérapeutique ou prophylactique, d'un agent, qui :
a) inhibe la voie classique du complément, la voie alterne du complément, et la voie des lectines du complément ; et/ou
b) inhibe l'activité des eicosanoïdes ;
pour une utilisation dans le traitement ou la prévention des effets inflammatoires d'une infection virale du tractus respiratoire ;
où l'agent est :
(i) une protéine comprenant ou étant constituée des acides aminés 19 à 168 ou des acides aminés 1 à 168 de la séquence d'acides aminés SEQ ID n° 2 ;
(ii) un homologue d'une protéine telle que définie dans (i) comprenant ou étant constitué d'une séquence ayant au moins 90% d'identité avec la séquence des acides aminés 19 à 168 ou des acides aminés 1 à 168 de la séquence d'acides aminés SEQ ID n° 2 ; ou
(iii) un fragment d'une protéine telle que définie dans (i) ci-dessus ou d'un homologue tel que défini dans (ii) ci-dessus, où le fragment fixe le complément C5 et/ou LTB4.

2. Agent pour une utilisation selon la revendication 1, où l'agent fixe le complément C5.

3. Agent pour une utilisation selon la revendication 1, où l'agent inhibe le clivage du complément C5 par la C5 convertase en complément C5a et en complément C5b-9 (MAC) .

4. Agent pour une utilisation selon l'une quelconque des revendications 1 à 3, où l'agent fixe LTB4.

5. Agent pour une utilisation selon l'une quelconque des revendications 1 à 4, où l'agent est dérivé d'un arthropode hématophage.

6. Agent pour une utilisation selon l'une quelconque des revendications 1 à 5, où l'agent est administré sous forme d'une molécule d'acide nucléique codant pour une protéine, un homologue ou un fragment tels que cités selon la revendication 1.

7. Agent pour une utilisation selon la revendication 6, où la molécule d'acide nucléique comprend ou est constituée des bases 55 à 507 de la séquence nucléotidique SEQ ID n° 1.

8. Agent pour une utilisation selon la revendication 7, où la molécule d'acide nucléique comprend ou est constituée des bases 1 à 507 de la séquence nucléotidique SEQ ID n° 1.

9. Agent pour une utilisation selon l'une quelconque des revendications 1 à 8, où le sujet est un mammifère, préférablement un être humain.

10. Agent pour une utilisation selon l'une quelconque des revendications 4 à 9, où l'agent est administré selon une dose suffisante pour fixer autant de C5 et/ou LTB4 disponibles que possible chez le sujet, plus préférablement tous les C5 disponibles.

11. Agent pour une utilisation selon l'une quelconque des revendications 1 à 10, où l'effet inflammatoire d'une infection virale du tractus respiratoire est une insuffisance respiratoire, telle qu'une lésion pulmonaire aiguë ou un syndrome de détresse respiratoire aigu, ou les séquelles d'une insuffisance respiratoire, y compris un syndrome de défaillance multiviscérale.

12. Agent pour une utilisation selon l'une quelconque des revendications 1 à 11, où l'infection virale est une infection des tractus respiratoires supérieur ou inférieur, y compris l'influenza virus pandémique, tel que le virus de la grippe A H5N1 (grippe aviaire) et de la grippe A H1N1 (grippe porcine), et le coronavirus du SRAG.
